# EUROPÄISCHE PATENTSCHRIFT

(11) **EP 2 435 408 B1**
(45) Veröffentlichungstag und Bekanntmachung des Hinweises auf die Patenterteilung: **07.01.2015**
(21) Anmeldenummer: 10718088.7
(22) Anmeldetag: 05.05.2010
(51) Int. Cl.: C07D 213/75

(54) **VERFAHREN ZUR HERSTELLUNG VON FLUPIRTIN**
METHOD FOR PRODUCING FLUPIRTINE
PROCÉDÉ DE FABRICATION DE FLUPIRTINE

(30) Priorität: 29.05.2009 DE 102009023162
(43) Veröffentlichungstag der Anmeldung: 04.04.2012
(73) Patentinhaber: Corden Pharma International GmbH, 68723 Plankstadt (DE)
(72) Erfinder: JAS, Gerhard, 12249 Berlin (DE); FREIFELD, Ilia, 61118 Bad Vibel (DE)
(74) Vertreter: Wagner, Jutta
(86) Internationale Anmeldenummer: PCT/EP2010/002747
(87) Internationale Veröffentlichungsnummer: WO 2010/136113

(56) Entgegenhaltungen:
- EP-A2- 0 199 951
- WO-A1-98/47872
- SCHWOCH S ET AL: "2,3-DIHYDROSPIRO[1H-4- AND 5-AZABENZIMIDAZOLE-2.1'-CYCLOHEXANE] (= SPIRO[CYCLOHEXANE-1,2'(3'H)-1'H-IMIDAZO[4, 5-B] PYRIDINE ] AND SPIRO[CYCLOHEXANE-1,2'(3'H)-1'H-IMIDAZO[4, 5-C] PYRIDINE] ): REACTIONS WITH NUCLEOPHILES" HELVETICA CHIMICA ACTA, VERLAG HELVETICA CHIMICA ACTA, BASEL, CH LNKD- DOI:10.1002/HLCA.19940770811, Bd. 77, Nr. 8, 1. Januar 1994 (1994-01-01) , Seiten 2175-2190, XP002073789 ISSN: 0018-019X

## Beschreibung

Die vorliegende Erfindung betrifft ein Verfahren zur Herstellung von Flupirtin, insbesondere von Flupirtin-Maleat.

Flupirtin-Maleat ist der INN für 2-Amino-3-ethylcarbamato-6-(4-fluoro-benzylamino)-pyridin-maleat, CAS: 75507-68-5, Molmasse 420,40 g/mol, Summenformel C₁₅H₁₇FN₄O₂ • C₄H₄O₄, und entspricht der Struktur in Formel I.

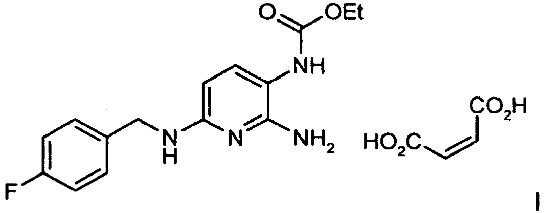

Flupirtin-Maleat wird z.B. unter dem Handelsnamen Katadolon® als Analgetikum eingesetzt.

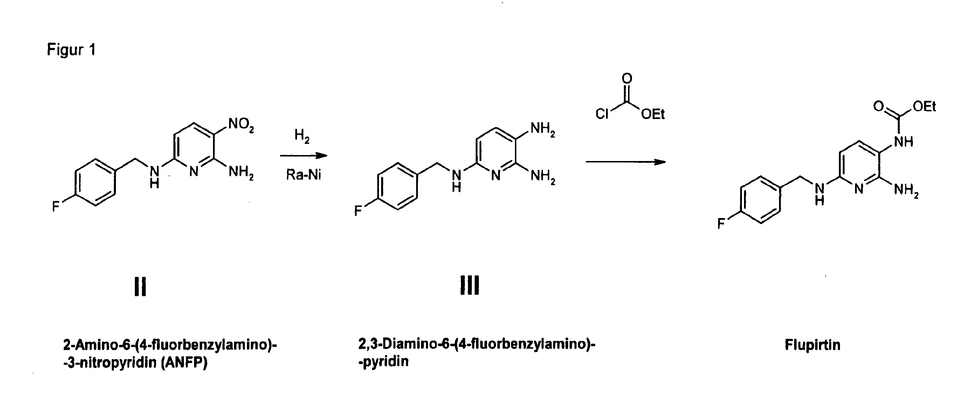

Die DE 31 33 519 beschreibt ein Verfahren zur Herstellung von Flupirtin-Maleat als Gemisch von polymorphen Formen A und B, wobei A mit einem Anteil > 60 % vorliegt. Die zentralen Reaktionsschritte sind dabei die in Figur 1 gezeigte Hydrierung von 2-Amino-6-(4-fluorbenzylamino)-3-nitropyridin (Formel II), im folgenden auch als ANFP bezeichnet, mittels Ra-Nickel zum 2,3-Diamino-6-(4-fluorbenzylamino)-pyridin (Formel III), und die anschließende regioselektive Acylierung mit Chlorameisensäureester zur freien Flupirtin-Base. Durch die Fällung als Maleat sollen blaue Verunreinigungen, die bei der Herstellung des HCl-Salzes anfallen, beseitigt werden. Die Aufreinigung des Flupirtin-Maleat wird durch Freisetzung der Base aus dem Maleat, Behandlung mit Aktivkohle und erneute Fällung als Maleat erzielt. Trotz dieser langwierigen und ökonomisch teuren Aufreinigungsstrategie können Spuren an farblichen Verunreinigungen nur schwer entfernt werden.

In WO 98/47872 wird ein Verfahren zur Herstellung von Flupirtin Maleat beschrieben, bei dem in wasserlöslichen Alkoholen (IPA) gearbeitet wird. Es werden drei Varianten vorgeschlagen. Variante 1 beinhaltet eine Umsetzung von ANFP an Ra-Nickel in IPA an die sich direkt die Acylierung anschließt und die Fällung eines als Rohmaleat bezeichneten Produkts durch "sehr raschen" Absaugprozess in eine wässrige Maleinsäurelösung. Es fällt ein gefärbtes Rohmaleat an, welches durch Umkristallisation aus Isopropanol/Wasser gereinigt werden soll. Die Nachstellung dieser Variante im Labor ergab jedoch ein gefärbtes Produkt. Gemäß Variante 2 soll ein durch Absaugen in 50 bis 60 °C warme Maleinsäurelösung erhaltenes Rohmaleat bereits farblos sein. Dies ließ sich ebenfalls nicht bestätigen. Gemäß der dritten Variante wird die nach der Acylierung gebildete Flupirtinbase nicht in situ umgesetzt sondern in Ethanol oder Wasser gefällt und umkristallisiert, bevor die weitere Umsetzung mit Maleinsäure erfolgt. Auch mit den in dieser Schrift genannten Verfahren ist ein reinweißes Flupirtin-Maleat nicht ohne weiteres erhältlich.

Aus EP 199 951 ist die Herstellung von 2-Amino-6-(4-fluorbenzylamino)-3-nitropyridin mittels Umsetzung von 2-Amino-3-nitro-6-methoxypyridin mit 4-Fluorbenzylamin bekannt, wobei dort auch auf die Möglichkeit der Reduzierung der Nitrogruppe und Einführung einer Carbethoxygruppe hingewiesen wird.

Aus Schwoch S. et al, "2,3-Dihydroxyspiro[1H-4- and 5-azabezimidazol-2,1'-cyclohexane] (=Spiro[cyclohexane-1,2'(3'H)-1'H-imidazo[4,5-b[pyridine] and Spiro[cyclohexane-1,2'(3'H)-1'H-imidazo[4,5-c[pyridine]): Reactions with Nucleophils", Helvetica Chimica Acta, Vol. 77 (1994) Seite 2181 ist eine alternative Route zur Herstellung von Flupirtinmaleat bekannt, welche über eine Umsetzung der genannten Spiroverbindungen mit Nukleophilen und anschließende Reduktion mit Na₂S₂O₄ verläuft.

Bei den bisher beschriebenen Verfahren ist das Auftreten von intensiv gefärbten Verunreinigungen, die eine Aufreinigung des Wirkstoffes erschweren, ein großer Nachteil. Ihren Ursprung haben diese Verunreinigungen vermutlich im Hydrierungsprozess. Bisher ist nur die Behandlung des Endproduktes mit Aktivkohle als einigermaßen erfolgreiches Verfahren zur Entfernung der Verunreinigungen bekannt. Dieses Vorgehen wird im Hinblick auf die Verluste bei der Gesamtausbeute bei einem Wirkstoff auf der letzten Herstellungsstufe nur ungern akzeptiert und vermeidet im vorliegenden Fall farbige Verunreinigungen auch nicht vollständig.

Aufgabe der vorliegenden Erfindung ist es daher, ein Verfahren zur Herstellung von Flupirtin-Säuresalzen, insbesondere Flupirtin-Maleat bereitzustellen, das, möglichst in höheren Ausbeuten, zu einem Material führt, das weniger, insbesondere keine, farblichen Verunreinigungen enthält. Wichtig ist eine leichtere Aufreinigung und speziell die Möglichkeit, gänzlich auf eine Aufreinigung mittels Aktivkohle zu verzichten.

Über die Natur der farbigen Verunreinigungen in den Verfahren nach dem Stand der Technik ist nichts bekannt, doch besteht die Vermutung, dass diese Verunreinigungen im wesentlichen bei der Hydrierung von ANFP gebildet werden. Es ist nicht klar, ob die bei der Hydrierung der Nitrogruppe durchlaufenen Oxidationsstufen dafür verantwortlich sind oder Metalikomplexe, die mit dem Hydrierungskatalysator gebildet werden können. Der Einfluss der Prozess- bzw. Hydrierungsparameter auf die Reinheit des Endproduktes ist in den genannten Patentanmeldungen oder in anderen Veröffentlichungen nur äußerst unzulänglich beschrieben.

Unter den bislang beschriebenen Hydrierungsbedingungen wird vermutlich ein Nebenprodukt gebildet, das aus der katalytischen Debenzylierung der Flupirtin-Vorstufe resultiert. Es ist bei der Verwendung von Ra-Nickel schwierig, den Gehalt dieser Verunreinigung im Produkt unter einen Schwellenwert von 0,1% zu drücken. Ein Gehalt von 0,2% dieser Verunreinigung im Rohprodukt führt dazu, dass eine einfache Umkristallisation zur Aufreinigung nicht mehr ausreicht. Die grünliche Färbung des Rohproduktes führen wir auf die Nickel-Salze zurück.

Überraschend wurde nun gefunden, dass bei einer Hydrierung mittels Palladium auf Aktivkohle, kurz Pd/C, anstelle des Ra-Nickel ein Flupirtin-Maleat erhalten wird, welches durch Umkristallisation gereinigt werden kann und damit ohne eine Aufreinigung mit Aktivkohle keine farbigen Verunreinigungen aufweist. Durch eine Veränderung der Prozessparameter unter Anwendung von Ra-Nickel ist dagegen eine solches Ergebnis praktisch nicht zu realisieren.

Erfindungsgemäß werden die obigen Probleme daher gelöst durch ein Verfahren zur Herstellung von Flupirtin-Säuresalzen, umfassend die Schritte:
a) Hydrierung von 2-Amino-6-(4-fluorbenzylamino)-3-nitropyridin mit Palladium auf Aktivkohle in einem inerten, mit Wasser mischbaren Lösungsmittel
b) Umsetzung des in a) erhaltenen 2,3-Diamino-6-(4-fluorbenzylamino)-pyridin zu Flupirtin durch Zugabe von Chlorameisensäureethylester und einer Base
c) Filtration
d) Versetzen des Filtrats aus Schritt c) mit einer Lösung einer Säure oder eines Säuresalzes in Wasser
e) Fällung des Flupirtin-Säuresalzes und Abfiltrieren.

Vorzugsweise wird das abfiltrierte Flupirtin-Säuresalz getrocknet.

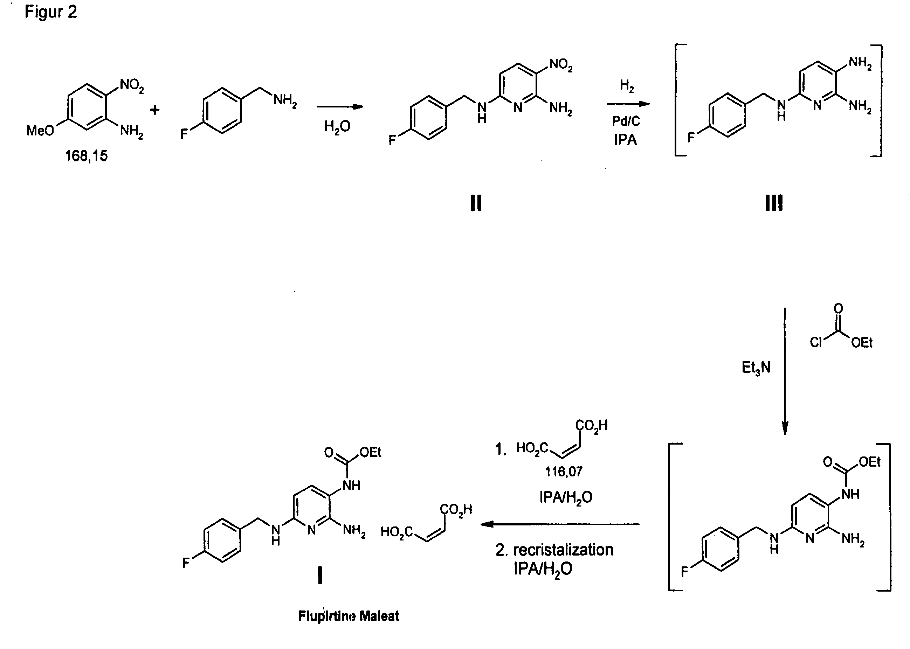

Das Flupirtin-Säuresalz kann durch Umkristallisieren aus einem Wasser/Lösungsmittelgemisch noch weiter aufgereinigt werden. Die Reaktionsschritte bei dem erfindungsgemäßen Verfahren, einschließlich der Herstellung des Edukts 2-Amino-6-(4-fluorbenzylamino)-3-nitropyridin, sind in Figur 2 dargestellt.

Das erfindungsgemäße Verfahren bietet mehrere Vorteile:
- Die Ausbeute bei der Hydrierung mit Pd/C liegt um ca. 10% höher als bei der korrespondierenden Hydrierung mit Ra-Nickel.
- Im Gegensatz zu Ra-Nickel ist der bei der Hydrierung verwendete Pd/C-Katalysator regenerierbar und liefert somit einen ökonomischen Vorteil.
- Gefärbte Verunreinigungen fallen in deutlich geringerem Umfang an und sind sehr leicht zu entfernen. Nach der 1. Fällung wird ein grünlich-weißes Material erhalten, das nach einer anschließenden Umkristallisation komplett frei von farblichen Verunreinigungen ist und eine weiße Farbe aufweist.
- Das bei der Hydrierung mit Ra-Nickel anfaillende Nebenprodukt kann bei der Reduktion mit Pd/C viel leichter beherrscht werden und fällt in geringeren Mengen an. Im Falle von Flupirtin-Maleat ist es bereits nach der 1. Umkristallisation nur noch mit einem Gehalt von deutlich < 0,1% nachweisbar und die Reinheit des Flupirtin-Maleats beträgt > 99,8%.

Erfindungsgemäß wird insbesondere Flupirtin-Maleat als Flupirtin-Säuresalz hergestellt. Es ist jedoch mit dem erfindungsgemäßen Verfahren möglich, durch Umsetzung mit der entsprechenden Säure oder einem geeigneten Säuresalz jegliche Säuresalze der Flupirtin-Base herzustellen. Bevorzugt werden Flupirtin-Säuresalze von physiologisch verträglichen Säuren hergestellt, z.B. von Salzsäure, Schwefelsäure oder organischen Säuren wie Essigsäure, Milchsäure, Äpfelsäure, Weinsäure, Salicylsäure, Fumarsäure usw., ganz besonders bevorzugt von Maleinsäure.

Der erste Schritt des erfindungsgemäßen Verfahrens ist die Hydrierung von 2-Amino-6-(4-fluorbenzylamino)-3-nitropyridin. Diese erfolgt erfindungsgemäß an Palladium auf Aktivkohle, im folgenden auch als Pd/C bezeichnet. Dazu wird das 2-Amino-6-(4-fluorbenzylamino)-3-nitropyridin zunächst in einem unter den Bedingungen der Hydrierung inerten Lösungsmittel gelöst. Das Lösungsmittel muss unter den Bedingungen der Hydrierung inert sein, d.h. es darf nicht selber hydriert werden oder anderweitig reagieren. Als Lösungsmittel eignen sich beispielsweise Alkohole, Ether, Ester usw. besonders aber Alkohole, insbesondere Ethanol, n-Propanol, Isopropanol, n-Butanol, sek-Butyalkohnol und tert-Butylalkohol, ganz besonders bevorzugt Isopropanol. Wasserlöslich oder mit Wasser mischbar heißt, dass das Lösungsmittel zumindest im Bereich der Volumenverhältnisse von 10:90 bis 90:10, vorzugsweise im Bereich von 5:95 bis 95:5 und insbesondere in jedem Verhältnis mit Wasser mischbar ist.

Die Hydrierung erfolgt insbesondere unter einem erhöhten Druck, beispielsweise von 1 bis 10 bar, vorzugsweise von 3 bis 5 bar Wasserstoff. Die Temperatur sollte im Bereich von 40 bis 100 °C, vorzugsweise von 60 bis 80 °C und besonders bevorzugt bei etwa 70 °C liegen. Pro g 2-Amino-6-(4-fluorbenzyl-amino)-3-nitropyridin werden bevorzugt 0,01 bis 0,1 g Katalysator, insbesondere 0,04 bis 0,06 g Katalysator verwendet, berechnet für 5 % Pd/C mit 50 % Wasser.

Typischerweise benötigt die Hydrierung eine Reaktionszeit von 7 bis 9 Stunden, insbesondere von 7,5 bis 8,5 Stunden. Eine Hydrierungszeit von mehr als 12 Stunden ist zu vermeiden, da dabei stark gefärbte Verunreinigungen entstehen und auch die Gesamtausbeute sinkt. Weiterhin ist üblicherweise nach ca. 6 Stunden ein Temperaturanstieg, typischerweise um etwa 0,4 °C, zu beobachten. Nach diesem Temperaturanstieg sollte noch mindestens 1 Stunde nachgerührt werden, ansonsten verringert sich die Gesamtausbeute um ca. 10 %.

Nach Abschluss der Hydrierung kann das erhaltene 2,3-Diamino-6-(4-fluorbenzylamino)-pyridin gewünschtenfalls isoliert werden, vorzugsweise wird es ohne Isolierung zum Flupirtin umgesetzt. Dazu wird Chlorameisensäureethylester zugesetzt. Mengen von 1,1 bis 1,3 mol Chlorameisensäureethylester pro mol 2,3-Diamino-6-(4-fluorbenzylamino)-pyridin haben sich bewährt. Es liegt im Bereich der Erfindung auch andere Acylierungsmittel einzusetzen, z.B. Bromide. Gleichzeitig oder anschließend setzt man eine Base zu, geeignet sind z.B. Trialkylamine, besonders Triethylamin. Hier beträgt die Menge vorzugsweise von 2 bis 1,4 mol pro mol 2,3-Diamino-6-(4-fluorbenzylamino)-pyridin. Es hat sich bewährt, die Temperatur bei der Zugabe auf 35 bis 60 °C, insbesondere 40 bis 50 °C zu halten. Vorzugsweise wird noch 0,5 bis 5 Stunden, insbesondere 1 bis 2 Stunden nachgerührt, wobei die Temperatur vorzugsweise etwas höher gehalten wird, z.B. 5 bis 15 °C, insbesondere 10 °C höher als bei der Zugabe.

Die in Schritt b) anfallenden Salze werden abfiltriert. Das in Schritt c) dabei erhaltene Filtrat enthält Flupirtin in dem gewählten inerten Lösungsmittel. Diese Lösung wird mit einer, vorzugsweise wässrigen, Lösung der gewünschten Säure oder eines ihrer Salze vermischt. Insbesondere wird das Filtrat zu der Lösung der Säure bzw. des Säuresalzes zugegeben. Vorzugsweise wird die Säure verwendet. Mengen im Bereich von 1,5 bis 1,7 mol Säure(salz) pro mol Flupirtin haben sich als besonders brauchbar erwiesen, wobei man bei der Berechnung zweckmäßig auf ANFP (d.h die theoretisch mögliche Menge Flupirtin) bezieht, da das Flupirtin vorzugsweise nicht isoliert wird. Bei der Vermischung fällt das Flupirtin-Säuresalz aus. Zur Vervollständigung der Umsetzung und Fällung wird die Suspension noch 0,5 bis 5 Stunden, vorzugsweise 1 bis 3 Stunden und insbesondere etwa 2 Stunden gerührt.

Zur Isolierung wird das Flupirtin-Säuresalz abfiltriert und üblicherweise der Filterkuchen mit Wasser und/oder dem Lösungsmittel nachgewaschen.

Zur weiteren Reinigung kann das Flupirtin-Säuresalz umkristallisiert oder auf eine andere an sich bekannte Weise aufgereinigt werden. Vorteilhaft ist, dass das erfindungsgemäße erhaltenen Flupirtin-Säuresalz bereits sehr rein ist und daher keine Aufreinigung mit Aktivkohle benötigt.

Überraschend wurde gefunden, dass eine besonders effektive Reinigung des Flupirtin-Säuresalzes durch Umkristallisation aus einem Isopropanol/Wasser-Gemisch möglich ist.

Die Umkristallisation erfolgt, indem man das Flupirtin-Säuresalz in einem Gemisch von Isopropanol und Wasser suspendiert, die Suspension bis zum Rückfluss erhitzt, wobei das Flupirtin-Säursalz sich löst, und die Lösung abkühlen lässt. Das Gemisch sollte Isopropanol und Wasser in einem Masseverhältnis von 18:1 bis 3:1, vorzugsweise von 15:1 bis 4:1, und insbesondere von 13:1 bis 5:1 enthalten. In der Regel wird während der Abkühlphase gerührt. Vorzugsweise wird nach dem Abkühlen noch einige Zeit weiter gerührt, beispielsweise 10 bis 60 Minuten. Danach wird das Flupirtin-Säuresalz durch Filtration abgetrennt, gewünschtenfalls nachgewaschen und/oder getrocknet.

Die Reinheit des erfindungsgemäß hergestellten Flupirtin-Säuresalzes beträgt typischerweise nach Schritt e) 99,5 bis 99,7 % und nach einer einmaligen Umkristallisation 99,8 % oder mehr. Es sind insbesondere keine färbenden Verunreinigungen enthalten, d.h. es handelt sich um hochreines, weißes Flupirtin-Säuresalz, ohne dass eine Aufreinigung an Aktivkohle notwendig wäre.

Die Erfindung soll anhand der folgenden Beispiele erläutert werden, ohne jedoch auf die speziell beschriebenen Ausführungsformen beschränkt zu sein. Soweit nichts anderes angegeben ist oder sich aus dem Zusammenhang zwingend anders ergibt, beziehen sich Prozentangaben auf das Gewicht, im Zweifel auf das Gesamtgewicht der Mischung.

### Beispiel 1: Herstellung von 2-Amino-6-(4-fluorobenzylamino)-3-nitropyridin

170 g 2-Amino-6-methoxy-3-nitropyridin und 196 g Wasser wurden vorgelegt. 194 g 4-Fluorobenzylamin wurden zugegeben und die Reaktionsmischung wurde unter Rückfluss gerührt. Nach der Beendigung der Reaktion wurde auf Raumtemperatur abgekühlt, der Niederschlag wurde abfiltriert und mit Wasser nachgewaschen. Das Feuchtprodukt wurde in Wasser suspendiert und mit 30 g Salzsäure (37%) bis pH = 1 versetzt. Der Niederschlag wurde abfiltriert und mit Wasser gewaschen. Getrocknetes Rohprodukt (242 g, 95% HPLC-Reinheit) wurde in 465 ml Toluol 1 Stunde lang bei 90 °C gerührt. Die entstandene Suspension wurde heiß filtriert und der Filterkuchen mit Toluol nachgewaschen. 220 g 2-Amino-6-(4-fluorobenzylamino)-3-nitropyridin wurden als gelber Feststoff (99.5% HPLC-Reinheit) erhalten. Die Ausbeute betrug 85%.

### Beispiel 2: Herstellung von Flupirtin Maleat an Pd/C

50 g 2-Amino-6-(4-fluorobenzylamino)-3-nitropyridin, 2.5 g Palladium auf Aktivkohle und 267 g Isopropanol wurden mit Wasserstoff bei 4.5 bar und 70 °C hydriert. Nach der Beendigung der Reaktion wurden 20.2 g Chlorameisensäureethylester, 24.8 g Triethylamin und 4.96 g Chlorameisensäureethylester bei 45 °C zugegeben. Danach wurde die Reaktionsmischung 0.5 h lang bei 55 °C nachgerührt. Anschließend wurde bei Raumtemperatur filtriert. Das Filtrat wurde dann zu einer Lösung von 35.6 g Maleinsäure in 1100 g Wasser bei Raumtemperatur zugegeben. Die entstandene Suspension wurde 2 h lang bei Raumtemperatur nachgerührt. Der Niederschlag wurde abfiltriert und mit Wasser und Isopropanol nachgewaschen. Der getrocknete Filterkuchen (99.6% HPLC-Reinheit) wurde in 842 g Isopropanol und 158 g Wasser suspendiert und zum Rückfluss erhitzt. Die entstandene klare Lösung wurde auf Raumtemperatur abgekühlt und bei Raumtemperatur nachgerührt. Der Niederschlag wurde abfiltriert und mit Isopropanol/Wasser-Mischung nachgewaschen. Der Filterkuchen wurde bei 50 °C getrocknet. 61 g Flupirtin Maleat (HPLC-Reinheit > 99.8%) wurden als weißer Feststoff erhalten. Die Ausbeute betrug 77%.

**Vergleichsbeispiel 3: Herstellung von Flupirtin Maleat** 50 g 2-Amino-6-(4-fluorobenzylamino)-3-nitropyridin, 2.5 g Palladium auf Aktivkohle und 267 g Isopropanol wurden mit Wasserstoff bei 4.5 bar und 70 °C hydriert. Nach der Beendigung der Reaktion wurde zusätzlich 8 h lang bei 70 °C hydriert. Danach wurden 20.2 g Chlorameisensäureethylester, 24.8 g Triethylamin und 4.96 g Chlorameisensäureethylester bei 20 °C zugegeben. Danach wurde die Reaktionsmischung 1.5 h lang bei 55 °C nachgerührt. Anschließend wurde bei Raumtemperatur filtriert. Das Filtrat wurde dann zu einer Lösung von 35.6 g Maleinsäure in 1000 g Wasser bei Raumtemperatur langsam zugegeben. Die entstandene Suspension wurde 1 h lang bei Raumtemperatur nachgerührt. Der Niederschlag wurde abfiltriert und mit Wasser und Isopropanol nachgewaschen. Getrockneter Filterkuchen (HPLC-Reinheit 91.5%) wurde in 828 g Isopropanol/Wasser-Gemisch (Masseverhältnis 5,3:1) suspendiert und auf 70 °C erhitzt. Die entstandene klare Lösung wurde auf Raumtemperatur abgekühlt und bei Raumtemperatur nachgerührt. Der Niederschlag wurde abfiltriert und mit isopropanol/Wasser-Gemisch nachgewaschen. Der Filterkuchen wurde bei 50 °C getrocknet. 43 g Flupirtin Maleat (HPLC-Reinheit 97.8%) wurden als weiß-grauer Feststoff erhalten. Die Ausbeute betrug 55%.

### Vergleichsbeispiel 4: Herstellung mit Ra-Nickel

### Vergleichsbeispiel 4a

25 g 2-Amino-6-(4-fluorobenzylamino)-3-nitropyridin, 1.72 g Raney-Nickel und 235 g Isopropanol wurden mit Wasserstoff bei 4.5 bar und 70 °C hydriert. Nach der Beendigung der Reaktion wurden 10.1 g Chlorameisensäureethylester, 12.4 g Triethylamin und 2.48 g Chlorameisensäureethylester bei 45 °C zugegeben. Danach wurde die Reaktionsmischung 0.5 h lang bei 55 °C nachgerührt. Anschließend wurde bei 50 °C unter Stickstoff-Atmosphäre filtriert. Das Filtrat wurde dann zu einer Lösung von 17.8 g Maleinsäure in 668 g Wasser bei 50 °C zugegeben. Danach wurde auf Raumtemperatur abgekühlt. Die entstandene Suspension wurde 1 h lang bei Raumtemperatur nachgerührt. Der Niederschlag wurde abfiltriert und mit Wasser und Isopropanol nachgewaschen. Getrockneter Filterkuchen (96.6% HPLC-Reinheit) wurde in 275 g Isopropanol und 52 g Wasser suspendiert und zum Rückfluss erhitzt. Die entstandene klare Lösung wurde auf Raumtemperatur abgekühlt und bei Raumtemperatur nachgerührt. Der Niederschlag wurde abfiltriert und mit Isopropanol nachgewaschen. Der Filterkuchen wurde bei 50 °C getrocknet. 18.5 g Flupirtin Maleat (HPLC-Reinheit 98.6%) wurden als weiß-blauer Feststoff erhalten. Die Ausbeute betrug 54%.

### Vergleichsbeispiel 4b:

25 g 2-Amino-6-(4-fluorobenzylamino)-3-nitropyridin, 1.72 g Raney-Nickel und 133 g Isopropanol wurden mit Wasserstoff bei 4.5 bar und 70 °C hydriert. Nach der Beendigung der Reaktion wurden 10.1 g Chlorameisensäureethylester, 12.4 g Triethylamin und 2.48 g Chlorameisensäureethylester bei 45 °C zugegeben. Danach wurde die Reaktionsmischung 1 h lang bei 55 °C nachgerührt. Anschließend wurde bei 20 °C unter Stickstoff-Atmosphäre filtriert. Das Filtrat wurde dann zu einer Lösung von 17.8 g Maleinsäure in 550 g Isopropanol bei Raumtemperatur langsam zugegeben. Die entstandene Suspension wurde 1 h lang bei Raumtemperatur nachgerührt. Der Niederschlag wurde abfiltriert und mit Isopropanol nachgewaschen. Der Filterkuchen wurde bei 50 °C getrocknet. 29.3 g Flupirtin-Maleat (HPLC-Reinheit 98.0%) wurden als dunkel-blauer Feststoff erhalten. Die Ausbeute betrug 73%.

### Vergleichsbeispiel 4c:

25 g 2-Amino-6-(4-fluorobenzylamino)-3-nitropyridin, 1.72 g Raney-Nickel und 133 g Isopropanol wurden mit Wasserstoff bei 4.5 bar und 70 °C hydriert. Nach der Beendigung der Reaktion wurden 10.1 g Chlorameisensäureethylester, 12.4 g Triethylamin und 2.48 g Chlorameisensäureethylester bei 45 °C zugegeben. Danach wurde die Reaktionsmischung 1 h lang bei 55 °C nachgerührt. Anschließend wurde bei 20 °C unter Stickstoff-Atmosphäre filtriert. Das Filtrat wurde dann zu einer Lösung von 17.8 g Maleinsäure in 700 g Wasser bei Raumtemperatur langsam zugegeben. Die entstandene Suspension wurde 1 h lang bei Raumtemperatur nachgerührt. Der Niederschlag wurde abfiltriert und mit Wasser nachgewaschen. Der Filterkuchen wurde bei 50 °C getrocknet. 29.6 g Flupirtin-Maleat (HPLC-Reinheit 96.8%) wurden als dunkel-blauer Feststoff erhalten. Die Ausbeute betrug 74%.

Den Vergleichsbeispielen 4a bis 4c lässt sich entnehmen, dass eine wirksame Reinigung eines mit Raney-Nickel hergestellten Produktes mit einer Umkristallisation nicht erreicht wird.

Die Erfindung bezieht sich auch auf sämtliche Kombinationen von bevorzugten Ausgestaltungen, soweit diese sich nicht gegenseitig ausschließen. Die Angaben "etwa" oder "ca." in Verbindung mit einer Zahlenangabe bedeuten, dass zumindest um 10 % höhere oder niedrigere Werte oder um 5 % höhere oder niedrigere Werte und in jedem Fall um 1 % höhere oder niedrigere Werte eingeschlossen sind.

## Patentansprüche

1. Verfahren zur Herstellung von Flupirtin-Säuresalz durch Hydrierung von 2-Amino-6-(4-fluorbenzylamino)-3-nitropyridin, Umsetzung des 2,3-Diamino-6-(4-fluorbenzylamino)-pyridin mit Chlorameisensäureester und Base zu Flupirtin, Filtration und Versetzen des Filtrats mit Säure oder Säuresalz zur Fällung des Flupirtin-Säuresalzes, **gekennzeichnet durch** die Schritte
a) Hydrierung von 2-Amino-6-(4-fluorbenzylamino)-3-nitropyridin mit Palladium auf Aktivkohle in einem inerten, mit Wasser mischbaren Lösungsmittel
b) Umsetzung des in a) erhaltenen 2,3-Diamino-6-(4-fluorbenzylamino)-pyridin zu Flupirtin **durch** Zugabe von Chlorameisensäureethylester und einer Base
c) Filtration
d) Versetzen des Filtrats aus Schritt c) mit einer Lösung einer Säure oder eines Säuresalzes in Wasser
e) Fällung des Flupirtin-Säuresalzes und Abfiltrieren.

2. Verfahren gemäß Anspruch 1, **dadurch gekennzeichnet, dass** als Säure oder Säuresalz in Schritt d) eine physiologisch verträgliche Säure, insbesondere eine organische physiologisch verträgliche Säure und besonders bevorzugt Maleinsäure oder ein Salz einer physiologisch verträglichen Säure, insbesondere eine organischen physiologisch verträglichen Säure und besonders bevorzugt von Maleinsäure eingesetzt wird.

3. Verfahren gemäß Anspruch 1 oder 2, **dadurch gekennzeichnet, dass** als inertes, wasserlösliches Lösungsmittel ein Alkohol, Ether und/oder Ester vorzugsweise ein Alkohol, bevorzugt eine Alkohol mit 2 bis 4 C-Atomen, und insbesondere Isopropanol verwendet wird.

4. Verfahren gemäß einem der Ansprüche 1 bis 3, **dadurch gekennzeichnet, dass** die Hydrierung unter einem erhöhtem Druck von 1 bis 10 bar, vorzugsweise von 3 bis 5 bar Wasserstoff erfolgt.

5. Verfahren gemäß einem der Ansprüche 1 bis 4, **dadurch gekennzeichnet, dass** die Hydrierung bei einer Temperatur im Bereich von 40 bis 100 °C, vorzugsweise von 60 bis 80 °C und besonders bevorzugt bei etwa 70 °C durchgeführt wird.

6. Verfahren gemäß einem der Ansprüche 1 bis 5, **dadurch gekennzeichnet, dass** eine Reaktionszeit von 7 bis 9 Stunden, insbesondere von 7,5 bis 8,5 Stunden für die Hydrierung eingehalten wird.

7. Verfahren gemäß einem der Ansprüche 1 bis 6, **dadurch gekennzeichnet, dass** in Schritt b) als Base ein Trialkylamin, insbesondere Triethylamin verwendet wird.

8. Verfahren gemäß einem der Ansprüche 1 bis 7, **dadurch gekennzeichnet, dass** die Temperatur bei der Zugabe in Schritt b) auf 35 bis 60 °C, insbesondere auf 40 bis 50 °C eingestellt wird.

9. Verfahren gemäß einem der Ansprüche 1 bis 8, **dadurch gekennzeichnet, dass** in Schritt b) nach der Zugabe noch 0,5 bis 5 Stunden, insbesondere 1 - 2 Stunden nachgerührt wird, wobei vorzugsweise die Temperatur 5 bis 15 °C, insbesondere 10 °C höher als bei der Zugabe gehalten wird.

10. Verfahren gemäß einem der Ansprüche 1 bis 9, **dadurch gekennzeichnet, dass** in Schritt d) und e) nach dem Versetzen des Filtrats mit der Säure oder dem Säuresalz noch 0,5 bis 5 Stunden, vorzugsweise 1 bis 3 Stunden und insbesondere etwa 2 Stunden gerührt wird.

11. Verfahren gemäß einem der Ansprüche 1 bis 10, **dadurch gekennzeichnet, dass** das Flupirtin-Säuresalz aus Schritt e) aus einem Isopropanol/Wasser Gemisch umkristallisiert wird.

12. Verfahren gemäß Anspruch 11, **dadurch gekennzeichnet, dass** die Umkristallisation erfolgt, indem man das Flupirtin-Säuresalz in einem Gemisch von Isopropanol und Wasser suspendiert, die Suspension bis zum Rückfluss erhitzt, wobei das Flupirtin-Säursalz sich löst, und die Lösung abkühlen lässt.

13. Verfahren gemäß Anspruch 11 oder 12, **dadurch gekennzeichnet, dass** das Gemisch Isopropanol und Wasser im Masseverhältnis von 18:1 bis 3:1, vorzugsweise von 15:1 bis 4:1 und insbesondere von 13:1 bis 5:1 enthält.

## Claims

1. Method for manufacturing flupirtine acid salt by hydrogenation of 2-amino-6-(4-fluorobenzyl amino)-3-nitropyridine, conversion of 2,3-diamino-6-(4-fluorobenzyl amino)-pyridine with chloroformate esters and base into flupirtine, filtration and mixing the filtrate with acid or acid salt for precipitating of the flupirtine acid salt, **characterised by** the steps
a) hydrogenation of 2-amino-6-(4-fluorobenzyl amino)-3-nitropyridine with palladium on activated carbon in an inert solvent that is miscible with water
b) converting of the 2,3-diamino-6-(4-fluorobenzyl amino)-pyridine obtained in a) into flupirtine by addition of ethyl chloroformate and a base
c) filtration
d) mixing the filtrate from step c) with a solution of an acid or an acid salt in water
e) precipitation of the flupirtine acid salt and filtering out.

2. Method according to claim 1, **characterised in that** a physiologically acceptable acid, in particular an organic physiologically acceptable acid, and particularly preferably maleic acid, or a salt of a physiologically acceptable acid, in particular an organic physiologically acceptable acid, and particularly preferably maleic acid, is used as the acid or acid salt in step d).

3. Method according to claim 1 or 2, **characterised in that** an alcohol, ether and/or ester, preferably an alcohol, preferably an alcohol having 2 to 4 C atoms, and in particular isopropanol, is used as the inert, water-soluble solvent.

4. Method according to anyone of claims 1 to 3, **characterised in that** the hydrogenation takes place at an increased pressure of 1 to 10 bar, preferably from 3 to 5 bar of hydrogen.

5. Method according to anyone of claims 1 to 4, **characterised in that** the hydrogenation is carried out at a temperature in the range from 40 to 100°C, preferably from 60 to 80°C, and particularly preferably at approximately 70°C.

6. Method according to anyone of claims 1 to 5, **characterised in that** a reaction time of 7 to 9 hours, in particular 7.5 to 8.5 hours, is observed for the hydrogenation.

7. Method according to anyone of claims 1 to 6, **characterised in that,** in step b), a trialkylamine, in particular triethylamine, is used as the base.

8. Method according to anyone of claims 1 to 7, **characterised in that** the temperature for the addition in step b) is set at 35 to 60°C, in particular at 40 to 50°C.

9. Method according to anyone of claims 1 to 8, **characterised in that,** in step b), after the addition, it is stirred for an additional 0.5 to 5 hours, in particular 1-2 hours, wherein the temperature is preferably maintained at 5 to 15°C, in particular 10°C, higher than for the addition.

10. Method according to anyone of claims 1 to 9, **characterised in that,** in step d) and e), after the mixing of the filtrate with the acid or the acid salt, it is stirred for an additional 0.5 to 5 hours, preferably 1 to 3 hours, and in particular approximately 2 hours.

11. Method according to anyone of claims 1 to 10, **characterised in that** the flupirtine acid salt from step e) is recrystallized from an isopropanol/water mixture.

12. Method according to claim 11, **characterised in that** the recrystallization occurs by the flupirtine acid salt being suspended in a mixture of isopropanol and water, the suspension being heated to reflux, wherein the flupirtine acid salt is dissolved and the solution is left to cool.

13. Method according to claim 11 or 12, **characterised in that** the mixture contains isopropanol and water in a mass ratio of 18:1 to 3:1, preferably 15:1 to 4:1, and in particular 13:1 to 5:1.

## Revendications

1. Procédé pour la préparation d'un sel d'acide de flupirtine par hydrogénation de la 2-amino-6-(4-fluorobenzylamino)-3-nitropyridine, mise en réaction de la 2,3-diamino-6-(4-fluorobenzylamino)-pyridine avec un ester de l'acide chloroformique et une base pour obtenir de la flupirtine, filtration du filtrat et addition à ce dernier d'un acide ou d'un sel d'acide à des fins de précipitation du sel d'acide de flupirtine, **caractérisé par** les étapes de :
a) hydrogénation de la 2-amino-6-(4-fluorobenzylamino)-3-nitropyridine avec du palladium sur du charbon activé dans un solvant inerte miscible à l'eau ;
b) mise en réaction de la 2,3-diamino-6-(4-fluorobenzyl-amino)-pyridine que l'on obtient à l'étape a) pour obtenir de la flupirtine par addition de l'ester éthylique de l'acide chloroformique et d'une base;
c) filtration;
d) addition au filtrat obtenu à l'étape c) d'une solution d'un acide ou d'un sel d'acide, dans de l'eau;
e) précipitation du sel d'acide de flupirtine et séparation par filtration.

2. Procédé selon la revendication 1, **caractérisé en ce qu'**on met en oeuvre à titre d'acide ou de sel d'acide à l'étape d) un acide physiologiquement acceptable, en particulier un acide organique physiologiquement acceptable et de manière particulièrement préférée, l'acide maléique ou un sel d'un acide physiologiquement acceptable, en particulier d'un acide organique physiologiquement acceptable et de manière particulièrement préférée de l'acide maléique.

3. Procédé selon la revendication 1 ou 2, **caractérisé en ce qu'**on utilise, à titre de solvant inerte soluble dans l'eau, un alcool, un éther et/ou un ester de manière préférentielle un alcool, de préférence un alcool contenant de 2 à 4 atomes de carbone, et en particulier l'isopropanol.

4. Procédé selon l'une quelconque des revendications 1 à 3, **caractérisé en ce que** l'hydrogénation a lieu sous une pression élevée de 1 à 10 bar, de préférence de 3 à 5 bar d'hydrogène.

5. Procédé selon l'une quelconque des revendications 1 à 4, **caractérisé en ce qu'**on effectue l'hydrogénation à une température dans la plage de 40 à 100 °C, de préférence de 60 à 80 °C et de manière particulièrement préférée à une température d'environ 70 °C.

6. Procédé selon l'une quelconque des revendications 1 à 5, **caractérisé en ce qu'**on respecte un temps de réaction de 7 à 9 heures, en particulier de 7,5 à 8,5 heures pour l'hydrogénation.

7. Procédé selon l'une quelconque des revendications 1 à 6, **caractérisé en ce qu'**on utilise comme base à l'étape b), une trialkylamine, en particulier la triéthylamine.

8. Procédé selon l'une quelconque des revendications 1 à 7, **caractérisé en ce qu'**on règle la température lors de l'addition à l'étape b), à une valeur de 35 à 60 °C, en particulier de 40 à 50 °C.

9. Procédé selon l'une quelconque des revendications 1 à 8, **caractérisé en ce qu'**on procède, à l'étape b), après l'addition, encore à une agitation ultérieure pendant un laps de temps de 0,5 à 5 heures, en particulier de 1 à 2 heures, la température étant de préférence maintenue à une valeur supérieure à raison de 5 à 15 °C, en particulier de 10 °C, à celle en vigueur lors de l'addition.

10. Procédé selon l'une quelconque des revendications 1 à 9, **caractérisé en ce que**, aux étapes d) et e), après l'addition au filtrat de l'acide ou du sel d'acide, on procède encore à une agitation pendant un laps de temps de 0,5 à 5 heures, de préférence de 1 à 3 heures et en particulier d'environ 2 heures.

11. Procédé selon l'une quelconque des revendications 1 à 10, **caractérisé en ce qu'**on recristallise le sel d'acide de flupirtine que l'on obtient à l'étape e) dans un mélange isopropanol/eau.

12. Procédé selon la revendication 11, **caractérisé en ce que** la recristallisation a lieu de telle sorte que l'on met en suspension le sel d'acide de flupirtine dans un mélange d'isopropanol et d'eau, on chauffe la suspension jusqu'au reflux, si bien que le sel d'acide de flupirtine se dissout, et on laisse refroidir la solution.

13. Procédé selon la revendication 11 ou 12, **caractérisé en ce que** le mélange contient de l'isopropanol et de l'eau dans le rapport en masse de 18:1 à 3:1, de préférence de 15:1 à 4:1, et en particulier de 13:1 à 5:1.
